Europäisches Patentamt

European Patent Office

Office européen des brevets

(19)

(11) Veröffentlichungsnummer: **0 207 451 B1**

(12) **EUROPÄISCHE PATENTSCHRIFT**

(45) Veröffentlichungstag der Patentschrift: **18.12.91**

(51) Int. Cl.5: **A61K 31/59**, A23K 1/00

(21) Anmeldenummer: 86108732.8

(22) Anmeldetag: 26.06.86

(54) **Pharmazeutische Präparate.**

(30) Priorität: **01.07.85 CH 2798/85**
**24.04.86 CH 1663/86**

(43) Veröffentlichungstag der Anmeldung:
**07.01.87 Patentblatt 87/02**

(45) Bekanntmachung des Hinweises auf die
Patenterteilung:
**18.12.91 Patentblatt 91/51**

(84) Benannte Vertragsstaaten:
**AT BE CH DE FR GB IT LI LU NL SE**

(56) Entgegenhaltungen:
**US-A- 4 225 596**

(73) Patentinhaber: **F. HOFFMANN-LA ROCHE AG**
**Postfach 3255**
**CH-4002 Basel(CH)**

(72) Erfinder: **Meier, Werner, Dr.**
**Bierastrasse 5**
**CH-4103 Bottmingen(CH)**
Erfinder: **Rambeck, Walter Albert, Dr.**
**Wertherstrasse 18**
**W-8000 München(DE)**
Erfinder: **Weiser, Harald, Dr.**
**248 Bürenweg**
**CH-4146 Hochwald(CH)**
Erfinder: **Zucker, Hermann, Prof. Dr.**
**Laplacestrasse 16**
**W-8000 München(DE)**

(74) Vertreter: **Lederer, Franz, Dr. et al**
**Lederer, Keller & Riederer Patentanwälte**
**Lucile-Grahn-Strasse 22**
**W-8000 München 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue pharmazeutische Präparate, die als Wirkstoffe hydroxylierte Derivate von Vitamin D enthalten. Insbesondere betrifft die Erfindung pharmazeutische Präparate enthaltend 1α,25-Dihydroxy-Vitamin $D_3$ (1,25(OH)$_2D_3$) im Gemisch mit 1α,24,25-Trihydroxy-Vitamin $D_3$ (1,24,25(OH)$_3$ $D_3$) oder 1α,25,26-Trihydroxy-Vitamin $D_3$ (1,25,26(OH)$_3$ $D_3$) oder 25,26-Dihydroxy-Vitamin $D_3$ (25,26 (OH)$_2$ $D_3$) oder 1α,24,25-Trihydroxy-Vitamin $D_3$ und 24,25-Dihydroxy-Vitamin $D_3$ (24,25 (OH)$_2$ $D_3$); oder 1α,25,26-Trihydroxy-Vitamin $D_3$ und 25,26-Dihydroxy-Vitamin $D_3$: und pharmazeutische Präparate enthaltend 1α-Hydroxy-Vitamin $D_3$ (1(OH)$D_3$) im Gemisch mit 1α,24,25-Trihydroxy-Vitamin $D_3$ oder 1α,25,26-Trihydroxy-Vitamin $D_3$; sowie übliche inerte pharmazeutische Hilfs- oder Trägerstoffe.

Die Erfindung umfasst somit die Wirkstoffkombinationen

1,25(OH)$_2$ $D_3$ + 1,24,25(OH)$_3$ $D_3$

1,25(OH)$_2$ $D_3$ + 1,25,26(OH)$_3$ $D_3$

1,25(OH)$_2$ $D_3$ + 25,26(OH)$_2$ $D_3$

1,25(OH)$_2$ $D_3$ + 24,25(OH)$_2$ $D_3$ + 1,24,25(OH)$_3$ $D_3$

1,25(OH)$_2$ $D_3$ + 25,26(OH)$_2$ $D_3$ + 1,25,26(OH)$_3$ $D_3$

1(OH) $D_3$ + 1,24,25(OH)$_3$ $D_3$

1(OH) $D_3$ + 1,25,26(OH)$_3$ $D_3$.


Die Erfindung betrifft weiterhin Futtermittel und Futtermittelzusätze, die die oben erwähnten Wirkstoffgemische enthalten, ferner die Verwendung dieser Wirkstoffgemische zur Herstellung von pharmazeutischen Präparaten, Futtermitteln und Futtermittelzusätzen.

Es ist bekannt, dass hydroxylierte Vitamin D-Derivate, wie das 1α-Hydroxy-Vitamin $D_3$, das 1α,25-Dihydroxy-Vitamin $D_3$ und das 1α,24,25-Trihydroxy-Vitamin $D_3$ zur Behandlung von Erkrankungen, die auf einem Mangel an Vitamin D oder dessen pharmakologisch wirksamen Metaboliten beruhen, verwendet werden können, vgl. US-A-4225596 Ueberraschend wurde gefunden, dass die Verabreichung der erfindungsgemässen Wirkstoffkombinationen im Vergleich zur Verabreichung der Einzelsubstanzen therapeutische Vorteile mit sich bringt. In einer bevorzugten Ausführungsform betrifft die Erfindung pharmazeutische Präparate enthaltend 1,24,25(OH)$_3D_3$ oder 1,25,26(OH)$_3$ $D_3$ im Gemisch mit 1,25(OH)$_2$ $D_3$. Ebenfalls bevorzugt ist die Kombination von 1,24,25(OH)$_3D_3$ mit 1(OH) $D_3$. Das 1,24,25-Trihydroxy-Vitamin $D_3$, vorzugsweise das 24R-Epimere, und das 1α,25,26-Trihydroxy-Vitamin $D_3$ verstärken in Konzentrationen, in denen sie allein kaum wirksam sind, die Wirkung von 1α-Hydroxy-Vitamin $D_3$ und 1α,25-Dihydroxy-Vitamin $D_3$. Dieser synergistische Effekt bleibt auch bei gestörter 1α-Hydroxylierung in den Nieren erhalten. Die Calciummobilisierung aus den Knochen geht bei Verabreichung der erfindungsgemässen Wirkstoffkombination gegenüber der Einzelverabreichung zurück, während gleichzeitig die Mineralisierung des Knochens zunimmt. Durch die kombinierte Verabreichung von 1α,24,25(bzw. 1α,25,26)-Trihydroxy-Vitamin $D_3$ mit 1α-Hydroxy- oder 1α,25-Dihydroxy-Vitamin $D_3$ können die letztgenannten Verbindungen niedriger als bei alleiniger Verabreichung dosiert werden, wodurch toxische Nebeneffekte vermieden oder verringert werden. Das Mengenverhältnis der Wirkstoffe in den erfindungsgemässen Präparaten ist im engeren Bereich nicht kritisch. Zweckmässig beträgt das Gewichtsverhältnis von 1α,24,25-Trihydroxy-Vitamin $D_3$ zu 1α-Hydroxy- oder 1α,25-Dihydroxy-Vitamin $D_3$ etwa 5:1 bis etwa 1:5. Bevorzugt ist ein Gewichtsverhältnis von etwa 1:1. Das Gewichtsverhältnis von 1,25(OH)$_2D_3$ zu 1,25,26(OH)$_3D_3$ kann zwischen 1:2 und 1:50 liegen, bevorzugt ist 1:10. Das Gewichtsverhältnis von 1,25(OH)$_2D_3$ zu 24,25(OH)$_2D_3$ oder 25,26(OH)$_2D_3$ kann 1:2 bis 1:20 betragen, bevorzugt ist 1:10. Die Wirksamkeit der erfindungsgemässen Wirkstoffkombinationen wird aus den nachstehend aufgeführten Versuchsresultaten ersichtlich.

I. Vitamin D-Test an wachsenden Hühnerküken

a) Kurativer Test

Die Präparate wurden männlichen Küken mit dem Futter verabreicht. Die Tiere erhielten in einer

2

Vorperiode während 8 Tagen ein rachitogenes, Vitamin D-freies Futter. In der anschliessenden Versuchsperiode erhielten die Tiere das gleiche Futter unter Zusatz der Wirkstoffe für die Dauer von 6 Tagen. Die Knochenanalytik wurde mit dem rechten Lauf (Phalangen I und II der Mittelzehe) durchgeführt. Das Knochentrockengewicht wurde nach 20 Minuten Kochen mit Wasser, Entfernung von Weichteilen, Sehnen und Knorpel und Trocknen bei 100° C bestimmt.

b) Prophylaktischer Test

Der Test wurde wie unter a) beschrieben durchgeführt, jedoch wurden die Wirkstoffe vom ersten Lebenstag an für die Dauer von 21 Tagen verabreicht.

Calcium-bindendes Protein (CaBP) wurde aus Schleimhauthomogenaten des Duodenums nach Corradino et al., Science 172, 731-3 (1971) gewonnen; die Bestimmung der spezifischen Aktivität erfolgte nach Wasserman et al. Science 152, 791-3 (1966) durch Zusatz von $^{45}$Ca in Gegenwart eines Kationenaustauschers und Bestimmung des in der Proteinlösung enthaltenen Prozentsatzes von $^{45}$Ca bezogen auf die gesamte Aktivität pro mg Protein. Die Aktivität der alkalischen Phosphatase im Serum wurde nach Bergmeyer, Methoden der enzymatischen Analyse, Bd I, 3. Auflage S. 888-892 bestimmt.

Die Resultate dieser Versuche sind in den Tabellen 1 bis 3 zusammengestellt. Die Vitamin D-Wirksamkeit zeigt sich in der Zunahme der CaBP-Aktivität und des Knochenascheanteils und in der Abnahme der alkalischen Phosphatase-Aktivität. Die ermittelten Werte zeigen, dass die erfindungsgemässe Wirkstoffkombination im Vergleich zu den einzeln verabreichten Komponenten eine mehr als additive Wirksamkeit ausübt.

In einer weiteren Versuchsreihe mit Küken wurde die 1α-Hydroxylase der Nieren nach Rambeck et al., Internat. J. Vit. Nutr. Res. 54, 25-34 (1984) durch Zusatz von Strontium zum Futter gehemmt. Die in Tabelle 4 angegebenen Versuchsergebnisse zeigen, dass der synergistische Effekt der erfindungsgemässen Wirkstoffkombination auch bei gestörter Nierenhydroxylierung beobachtet wird.

II. Knochencalcium-Mobilisierung am Hühnerküken

Hühnerküken erhielten während einer Woche eine calciumfreie Diät. Danach wurden die Testsubstanzen in 0,1 ml 75% Aethanol intravenös verabreicht. 12 Stunden danach wurden die Ca-Werte im Serum ermittelt. Die in Tabelle 5 aufgeführten Resultate zeigen, dass die Ca-Mobilisierung aus den Knochen bei Verabreichung der erfindungsgemässen Wirkstoffkombination nicht grösser ist als bei alleiniger Verabreichung der einzelnen Komponenten.

III. Eischalen-Test an der japanischen Wachtel

Dieser Test ermittelt die Vitamin D-Wirksamkeit anhand der Ca-Ausscheidung der Tiere über die Eischale. Der Test wurde nach Zucker et al., Naturwiss. 55, 447-455 (1968), durchgeführt. Die in Tabelle 6 angeführten Ergebnisse zeigen ebenfalls einen mehr als additiven Effekt der Wirkstoffkombination.

IV. Kurativer Vitamin-D-Test bei Ratten

Mit diesem Test wird die Vitamin D-Wirksamkeit aus dem Grad der Calcifizierung der Epiphysenfuge der Tibia ermittelt, wie von Weiser in "Das Tier im Experiment". Herausg. Wolf H. Weihe, Verlag Hans Huber, Bern-Stuttgart-Wien, 1978 beschrieben. Den Tieren wurde 1,25(OH)$_2$D$_3$ oder/und 1,24,25(OH)$_3$D$_3$ täglich während 7 Tagen mit der Schlundsonde, gelöst in Aethanol/Propylenglykol (1:10), verabreicht. Einen Tag später wurde die Calcifizierung der Epiphysenfuge röntgenographisch anhand einer 12-teiligen Skala bewertet. Mit der Wirkstoffkombination wurde in dieser Testanordnung ein synergistischer Effekt erreicht (Tabelle 7).

## Tabelle 1

Vitamin D-Test an wachsenden Hühnerküken.

a) Kurativer Kükentest, 11 Tiere/Gruppe
Kombination von $1,25(OH)_2D_3$ und $1,24,25(OH)_3D_3$

| Diätzusatz pro kg | CaBP (spezifische Aktivität) | %Knochenasche bezogen auf Knochen-Trockenge-wicht (gepoolt) | alk.Phos-phatase (gepoolt) [Einheiten] |
|---|---|---|---|
| (neg. Kontrolle) | $1,87 \pm 0,40$ | 25,7 | 17 276 |
| $1,5$ µg $1,25 (OH)_2D_3$ | $3,69 \pm 1,58$ | 28,8 | 14 788 |
| $1,5$ µg $1,24,25(OH)_3D_3$ | $1,75 \pm 0,36$ | 26,3 | 15 293 |
| $3,0$ µg $1,24,25(OH)_3D_3$ | $2,31 \pm 0,40$ | 26,7 | 15 360 |
| $1,5$ µg $1,25(OH)_2D_3$ $+1,5$ µg $1,24,25(OH)_3D_3$ | $6,39 \pm 1,49$ | 30,2 | --- |
| $1,5$ µg $1,25(OH)_2D_3$ $+3,0$ µg $1,24,25(OH)_3D_3$ | $6,69 \pm 1,27$ | 31,0 | 11 293 |

## Tabelle 2

### Vitamin D-Test an wachsenden Hühnerküken

b) prophylaktischer Test, 10 Tiere/Gruppe
Kombination von $1,25(OH)_2 D_3$ und $1,25,26(OH)_2 D_3$ bzw. $1,25,26(OH)_3 D_3$

| Diätzusatz pro kg | CaBP (spezif. Aktivität) | %Knochenasche bezogen auf Knochen-Trockengewicht (gepoolt) | alk.Phosphatase (gepoolt) [Einheiten] | Ca im Serum [mg/ 100 ml] | Gewichtszunahme [g] |
|---|---|---|---|---|---|
| (negative Kontrolle) | 1,0 | 22,9 | 23 615 | 5,91 | 161 |
| 8 µg Vitamin $D_3$ | 5,1 | 33,5 | 10 589 | 8,24 | 417 |
| 0,5 µg $1,25(OH_2)D_3$ | 1,5 | 25,8 | 21 633 | 6,17 | 262 |
| 1,0 µg $1,25(OH)_2D_3$ | 2,0 | 30,3 | 14 296 | 7,05 | 365 |
| 5 µg $1,25,26(OH)_3D_3$ | 1,6 | 25,8 | 17 220 | 7,21 | 254 |
| 15 µg $1,25,26(OH)_3D_3$ | 3,2 | 35,0 | 14 245 | 8,79 | 378 |
| 5 µg $25,26(OH)_2D_3$ | 1,6 | 24,2 | 17 473 | 6,73 | 260 |
| 15 µg $25,26(OH)_2D_3$ | 1,9 | 26,7 | 25 339 | 6,18 | 244 |
| 0,5 µg $1,25(OH)_2D_3$ + 5 µg $1,25,26(OH)_3D_3$ | 2,7 | 30,9 | 19 137 | 7,98 | 371 |

Tabelle 2 (Fortsetzung)

| Diätzusatz pro kg | CaBP (spezif. Aktivi-tät) | %Knochenasche bezogen auf Knochen-Trockenge-wicht (gepoolt) | alk.Phos-phatase (gepoolt) [Einheiten] | Ca im Serum [mg/ 100 ml] | Gewichts-zunahme [g] |
|---|---|---|---|---|---|
| 0,5 µg 1,25(OH)$_2$D$_3$ + 5 µg 25,26(OH)$_2$D$_3$ | 2,4 | 27,1 | 15 783 | 6,35 | 297 |
| 0,5 µg 1,25(OH)$_2$D$_3$ + 15 µg 25,26(OH)$_2$D$_3$ | 2,1 | 28,3 | 16 641 | 7,22 | 337 |
| 0,5 µg 1,25(OH)$_2$D$_3$ + 15 µg 1,25,26(OH)$_3$D$_3$ | 4,5 | 35,4 | 6 026 | 9,50 | 410 |

6

## Tabelle 3

<u>Vitamin D-Test an wachsenden Hühnerküken.</u>

(Kurativer Kükentest) 11 Tiere/Gruppe

Kombination von $1(OH)D_3$ und $1,24,25(OH)_3D_3$

| Diätzusatz pro kg | CaBP (spezifische Aktivität) | % Knochenasche vom Trockengewicht (gepoolt) |
|---|---|---|
| (neg. Kontrolle) | $1,43 \pm 0,46$ | 27,9 |
| $1,0\ \mu g\ 1(OH)D_3$ | $1,50 \pm 0,44$ | 29,2 |
| $1,5\ \mu g\ 1,24,25(OH)_3D_3$ | $1,68 \pm 0,35$ | 28,8 |
| $1,0\ \mu g\ 1(OH)D_3$ $+1,5\ \mu g\ 1,24,25(OH)_3D_3$ | $2,97 \pm 0,75$ | 31,0 |

## Tabelle 4

<u>Vitamin D-Test an wachsenden Hühnerküken.</u>
(kurativer Kükentest) Durch Strontium gehemmte $1\alpha$-Hydroxylierung, 10 Tiere/Gruppe

Kombination von $1,25(OH)_2D_3$ und $1,24,25(OH)_3D_3$

| Diätzusatz pro kg | CaBP (spezifische Aktivität) | % Knochenasche bezogen auf Knochen-Trockengewicht (gepoolt) |
|---|---|---|
| (neg. Kontrolle) | 1,54 | 20,1 |
| 3 µg $1,25(OH)_2D_3$ | 3,31 | 21,2 |
| 3 µg $1,24,25(OH)_3D_3$ | 1,57 | 19,6 |
| 3 µg $1,25(OH)_2D_3$ +3 µg $1,24,25(OH)_3D_3$ | 4,65 | 22,3 |

## Tabelle 5

Knochencalcium-Mobilisierung am Hühnerküken.

(1 Woche calciumfreie Diät)

| i.v. Gabe | mg Ca/100 ml Serum nach 12 Stunden |
|---|---|
| (neg. Kontrolle) | 4,6 |
| 0,013 µg 1,25(OH)$_2$D$_3$ | 4,8 |
| 0,025 µg " | 5,0 |
| 0,05 µg " | 5,3 |
| 0,125 µg " | 5,5 |
| 0,25 µg " | 5,9 |
| 1,25 µg " | 6,5 |
| 2,50 µg " | 7,4 |
| 0,05 µg 1,24,25(OH)$_3$D$_3$ | 5,4 |
| 0,25 µg " | 5,7 |
| 1,25 µg " | 6,3 |
| 0,05 µg 1,24,25(OH)$_3$D$_3$ +0,25 µg 1,25(OH)$_2$D$_3$ | 5,4 |
| 0,25 µg 1,24,25(OH)$_3$D$_3$ +0,25 µg 1,25(OH)$_2$D$_3$ | 5,8 |
| 1,25 µg 1,24,25(OH)$_3$D$_3$ +0,25 µg 1,25(OH)$_2$D$_3$ | 6,4 |

## Tabelle 6

Eischalentest an der japanischen Wachtel

12 Tiere/Gruppe. Kombination von $1,25(OH)_2D_3$ und $1,24,25(OH)_3D_3$

| Diätzusatz pro kg | Eischalentrockengewicht in mg pro Tier und Tag |
|---|---|
| (neg. Kontrolle) | 56 |
| $2,0$ µg Vitamin $D_3$ | 87 |
| $3,5$ µg Vitamin $D_3$ | 309 |
| $5,0$ µg Vitamin $D_3$ | 563 |
| $0,27$ µg $1,25(OH)_2D_3$ | 91 |
| $5,63$ µg $1,24,25(OH)_3D_3$ | 126 |
| $0,27$ µg $1,25(OH)_2D_3$ + $5,63$ µg $1,24,25(OH)_3D_3$ | 271 |

## Tabelle 7

### Kurativer Vitamin-D-Test an Ratten

(Röntgentest, 16 Tiere/Gruppe)

Kombination von $1,25(OH)_2D_3$ und $1,24,25(OH)_3D_3$

| Dosis pro Tier und Tag | Calcifizierung der Epiphysenfuge (Scoring-Werte ± SEM nach 7 Tagen) |
|---|---|
| Negative Kontrolle | 0 |
| $0,0203$ µg $1,25(OH)_2D_3$ | $4,4 \pm 0,21$ |
| $0,0105$ µg $1,24,25(OH)_3D_3$ | $1,8 \pm 0,21$ |
| $0,0210$ µg $1,24,25(OH)_3D_3$ | $3,7 \pm 0,18$ |
| $0,0203$ µg $1,25(OH)_2D_3$ plus $0,0105$ µg $1,24,25(OH)_3D_3$ | $7,8 \pm 0,32$ |
| $0,0203$ µg $1,25(OH)_2D_3$ plus $0,0210$ µg $1,24,25(OH)_3D_3$ | $9,8 \pm 0,21$ |

Die erfindungsgemässen Präparate sind dazu geeignet, den intestinalen Calciumtransport, den Calcium- und Phosphatspiegel im Serum, sowie die Einlagerung dieser Mineralien in den Knochen zu erhöhen. Diese Präparate können daher zur Behandlung von durch metabolische Calcium- und Phosphatmangelzustände gekennzeichneten Krankheiten Verwendung finden, insbesondere solchen, bei denen die Konzentration von endogen produziertem $1\alpha,25$-Dihydroxy Vitamin $D_3$ unter dem Normalwert liegt. Beispiele solcher Krankheiten sind Osteomalacie, Osteoporose, Rachitis, Osteitis fibrosa cystica, renale Osteodystrophie, Osteosclerose, Konvulsionen, Osteopenie, Fibrogenesis imperfecta ossium, sekundärer Hyperparathyroidismus, Hypoparathyroidismus, Cirrhose, obstruktive Gelbsucht, medulläres Karzinom, chronische renale Krankheiten, hypophosphatämische Vitamin D-resistente Rachitis, Vitamin D-abhängige Rachitis, Sarcoidose, Glucocorticoidantagonismus, Missabsorptionssyndrom, Steatorrhoe, tropische Sprue, idiopatische Hypercalcämie und Milchfieber. Weiterhin können die Präparate z.B. als Futterzusatz zur Verhütung der Dünnschaligkeit von Eiern, insbesondere Hühner- und Truthuhneiern, und zur Behandlung oder Verhütung von Beinschwäche (Knochenschwäche) beim Geflügel Verwendung finden.

Die erfindungsgemässen Präparate können beim Erwachsenen in Tagesdosen von etwa 0,01-1, vorzugsweise 0,05-0,2 Mikrogramm, bezogen auf $1,25(OH)_2D_3$ bzw. $1(OH)D_3$ bei den oben genannten Krankheiten verabreicht werden. Die erfindungsgemässen Präparate können oral, subcutan, intramuskulär, intravenös, intraperitoneal oder topisch appliziert werden. Beispiele von pharmazeutischen Präparaten sind Tabletten, Kapseln oder Elixiere zur oralen Verabreichung, und sterile Lösungen oder Suspensionen zur parenteralen Verabreichung. Diese pharmazeutischen Präparate können folgende Hilfsstoffe enthalten: Bindemittel, wie Tragantgummi, Akazie, Maisstärke oder Gelatine; Excipienten, wie Calciumphosphat; Sprengmittel, wie Mais oder Kartoffelstärke oder Alginsäure; Gleitmittel, wie Magnesiumstearat; Süsstoffe, wie Sucrose, Lactose oder Saccharin; Geschmacksmittel, wie Pfefferminze, Wintergrün oder Sherryöl. Es können andere Materialien, wie Ueberzüge zur Modifizierung des Aussehens der Präparate verwendet werden. Tabletten können z.B. mit Schellack und/oder Zucker überzogen werden. Ein Sirup oder Elixier kann die Aktivsubstanz. Sucrose als Süssmittel, Methyl- und Propylparaben als Konservierungsmittel, Farbstoffe und Geschmacksstoffe, wie Kirschen- oder Orangenaroma, enthalten.

Zur Behandlung des Milchfiebers bei trächtigen Wiederkäuern kommen Dosen von 0,2-1,5 mg/Tag, bezogen auf $1,25(OH)_2D_3$ bzw. $1(OH)D_3$ in den üblichen Verabreichungsformen in Betracht. Bei der Verabreichung an Geflügel kommen Dosierungen von 0,25-2,5 $\mu$g/kg Futter, bezogen auf $1,25(OH)_2D_3$ bzw. $1(OH)D_3$, oder entsprechend angepasste Mengen in der Tränke in Betracht.

Sterile Präparate zum Injizieren und/oder zur topischen Verabreichung können in üblicher Weise dadurch hergestellt werden, dass man die Wirkstoffe in einem Vehikel, wie einem 10-20% Aethanol-Wassergemisch, einem 10-20% Propylenglykol-Wassergemisch, einem in der Natur vorkommenden pflanzlicher Oel, wie Sesamöl, Erdnussöl, Baumwollsaatöl; oder einem synthetischen fetten Bindemittel, wie Aethyloleat, auflöst oder suspendiert.

Die folgenden Beispiele erläutern die Erfindung weiter.

Beispiel 1

Die Wirkstoffe können in einem pharmazeutisch verwendbaren Lösungsmittel wie Alkohol, Propylenglykol, Glycerin oder Polyäthylenglykol aufgelöst werden. Oberflächenaktive Mittel, wie Polyäthylenglykol, Sorbitanester, Dioctylnatriumsulfosuccinate, Polyoxyäthylen-polyoxypropylen-Copolymere, können zur Solubilisierung zugesetzt werden. Ein Konservierungsmittel zur Verhütung des mikrobiellen Wachstums kann auch zugesetzt werden. Beispiele von aus solchen Gemischen zusammensetzbaren Kapselformulierungen sind:

a)                                                                  pro Kapsel

1α,25-Dihydroxy-Vitamin D₃                    0,05 − 0,5 μg

1α,24,25-Trihydroxy-Vitamin D₃              0,05 − 0,5 μg

Polyäthylenglykol (PEG)                            400,0 mg

Butyliertes Hydroxy-

anisol (BHA)                                                 0,2 mg

Ascorbylpalmitat                                         1,0 mg

Die Wirkstoffe werden einer Lösung von BHA und Ascorbylpalmitat in PEG zugesetzt und das Gemisch wird unter einer Stickstoffatmosphäre aufgelöst. Die Flüssigkeit wird in Weichkapseln abgefüllt.

b)                                                                  pro Kapsel

1α,25-Dihydroxy-Vitamin D₃                    0,05 − 0,5 μg

1α,24,25-Trihydroxy-Vitamin D₃              0,05 − 0,5 μg

PEG 400 (oder PEG 6000)                         200,0 mg

Polyoxyäthylensorbitanmonooleat oder -monostearat

(Polysorbat 80 oder Polysorbat 60)                                                    200,0 mg

BHA                                                                0,2 mg

Ascorbylpalmitat                                         1,0 mg

Das Gemisch von PEG 6000 und Polysorbat 60 wird erhitzt, dann das BHA und das Ascorbylpalmitat zugesetzt und schliesslich werden die Wirkstoffe unter Stickstoff zugefügt. Das Gemisch wird in Hartkapseln

EP 0 207 451 B1

abgefüllt.

| c) | pro Kapsel |
|---|---|
| $1\alpha,25$-Dihydroxy-Vitamin $D_3$ | 0,05 – 0,5 µg |
| $1\alpha,25,26$-Trihydroxy-Vitamin $D_3$ | 0,5 – 5 µg |
| PEG 400 | 100,0 mg |
| PEG 4000 | 300,0 mg |
| BHA | 0,2 mg |
| Butyliertes Hydroxy-toluol (BHT) | 0,1 mg |
| Ascorbylpalmitat | 1,0 mg |

Das Gemisch von PEG 400 und PEG 4000 wird erhitzt, dann das BHT, BHA und das Ascorbylpalmitat zugesetzt und schliesslich werden die Wirkstoffe zugefügt und unter Stickstoff gelöst. Das Gemisch wird in Hartkapseln abgefüllt.

Beispiel 2

Einem nach den Normen für die Geflügelfütterung hergestellten Kükenalleinfutter werden pro kg Futter 0,25-2,5 µg $1\alpha,25$-Dihydroxy-Vitamin $D_3$ und 0,25-2,5 µg $1\alpha,24,25$-Trihydroxy-Vitamin $D_3$ zugemischt.

**Patentansprüche**

1. Pharmazeutische Präparate enthaltend $1\alpha,25$-Dihydroxy-Vitamin $D_3$ im Gemisch mit $1\alpha,24,25$-Trihydroxy-Vitamin $D_3$ oder $1\alpha,25,26$-Trihydroxy-Vitamin $D_3$ oder $25,26$-Dihydroxy-Vitamin $D_3$; oder $1\alpha,24,25$-Trihydroxy-Vitamin $D_3$ und $24,25$-Dihydroxy-Vitamin $D_3$; oder $1\alpha,25,26$-Trihydroxy-Vitamin $D_3$ und $25,26$-Dihydroxy-Vitamin $D_3$; und pharmazeutische Präparate enthaltend $1\alpha$-Hydroxy-Vitamin $D_3$ im Gemisch mit $1\alpha,24,25$-Trihydroxy-Vitamin $D_3$ oder $1\alpha,25,26$-Trihydroxy-Vitamin $D_3$; sowie übliche inerte pharmazeutische Hilfs- oder Trägerstoffe.

2. Präparate gemäss Anspruch 1, enthaltend $1\alpha,24,25$-Trihydroxy-Vitamin$D_3$ oder $1\alpha,25,26$-Trihydroxy-Vitamin $D_3$ im Gemisch mit $1\alpha,25$-Dihydroxy-Vitamin $D_3$.

3. Präparate gemäss Anspruch 1, enthaltend $1\alpha,24,25$-Trihydroxy-Vitamin$D_3$ im Gemisch mit $1\alpha$-Hydroxy-Vitamin $D_3$ oder $1\alpha,25$-Dihydroxy-Vitamin $D_3$.

4. Präparate gemäss Anspruch 3, in denen das Gewichtsverhältnis von $1\alpha,24,25$-Trihydroxy-Vitamin $D_3$ zu $1\alpha$-Hydroxy- oder $1\alpha,25$-Dihydroxy-Vitamin $D_3$ etwa 5:1 bis 1:5 beträgt.

5. Präparate gemäss Anspruch 4, in denen das Gewichtsverhältnis etwa 1:1 beträgt.

6. Präparate gemäss den Ansprüchen 1-5 enthaltend $1\alpha,24,25$-Trihydroxy-Vitamin $D_3$ und $1\alpha,25$-Dihydroxy-Vitamin $D_3$.

7. Präparate gemäss den Ansprüchen 1-6 enthaltend pro Dosiseinheit 0,05-0,5 µg von jedem Wirkstoff.

8. Futtermittel und Futtermittelzusätze enthaltend $1\alpha,25$-Dihydroxy-Vitamin $D_3$ im Gemisch mit $1\alpha,24,25$-Trihydroxy-Vitamin $D_3$ oder $1\alpha,25,26$-Trihydroxy-Vitamin $D_3$ oder $25,26$-Dihydroxy-Vitamin $D_3$; oder $1\alpha,24,25$-Trihydroxy-Vitamin $D_3$ und $24,25$-Dihydroxy-Vitamin $D_3$; oder $1\alpha,25,26$-Trihydroxy-Vitamin $D_3$ und $25,26$-Dihydroxy-Vitamin $D_3$; und Futtermittel und Futtermittelzusätze enthaltend $1\alpha$-Hydroxy-Vitamin $D_3$ im Gemisch mit $1\alpha,24,25$-Trihydroxy-Vitamin $D_3$ oder $1\alpha,25,26$-Trihydroxy-Vitamin $D_3$.

13

9. Verwendung von Gemischen von $1\alpha,25$-Dihydroxy-Vitamin $D_3$ mit $1\alpha,24,25$-Trihydroxy-Vitamin $D_3$ oder $1\alpha,25,26$-Trihydroxy-Vitamin $D_3$ oder $25,26$-Dihydroxy-Vitamin $D_3$; oder $1\alpha,24,25$-Trihydroxy-Vitamin $D_3$ und $24,25$-Dihydroxy-Vitamin $D_3$; oder $1\alpha,25,26$-Trihydroxy-Vitamin $D_3$ und $25,26$-Dihydroxy-Vitamin $D_3$; und Gemische von $1\alpha$-Hydroxy-Vitamin $D_3$ mit $1\alpha,24,25$-Trihydroxy-Vitamin $D_3$ oder $1\alpha,25,26$-Trihydroxy-Vitamin $D_3$ zur Herstellung von pharmazeutischen Präparaten, insbesondere solchen zur Behandlung und Prophylaxe von durch metabolische Calcium- und Phosphatmangelzustände gekennzeichneten Erkrankungen, und zur Herstellung von Futtermitteln oder Futtermittelzusätzen.

10. Verfahren zur Herstellung der pharmazeutischen Präparate, Futtermittel oder Futtermittelzusätze der Ansprüche 1-8 dadurch gekennzeichnet, dass man die Wirkstoffe mit üblichen inerten pharmazeutischen Hilfs- oder Trägerstoffen bzw. Futtermitteln oder in der Tiernahrung üblichen Trägerstoffen vermischt.

## Claims

1. Pharmaceutical preparations containing $1\alpha,25$-dihydroxy-vitamin $D_3$ in admixture with $1\alpha,24,25$-trihydroxy-vitamin $D_3$ or $1\alpha,25,26$-trihydroxy-vitamin $D_3$ or $25,26$-dihydroxy-vitamin $D_3$; or $1\alpha,24,25$-trihydroxy-vitamin $D_3$ and $24,25$-dihydroxy-vitamin $D_3$; or $1\alpha,25,26$-trihydroxy-vitamin $D_3$ and $25,26$-dihydroxy-vitamin $D_3$; and pharmaceutical preparations containing $1\alpha$-hydroxy-vitamin $D_3$ in admixture with $1\alpha,24,25$-trihydroxy-vitamin $D_3$ or $1\alpha,25,26$-trihydroxy-vitamin $D_3$; as well as usual inert pharmaceutical adjuvants or carrier materials.

2. Preparations in accordance with claim 1 containing $1\alpha,24,25$-trihydroxy-vitamin $D_3$ or $1\alpha,25,26$-trihydroxy-vitamin $D_3$ in admixture with $1\alpha,25$-dihydroxy-vitamin $D_3$

3. Preparations in accordance with claim 1 containing $1\alpha,24,25$-trihydroxy-vitamin $D_3$ in admixture with $1\alpha$-hydroxy-vitamin $D_3$ or $1\alpha,25$-dihydroxy-vitamin $D_3$.

4. Preparations in accordance with claim 3, in which the weight ration of $1\alpha,24,25$-trihydroxy-vitamin $D_3$ to $1\alpha$-hydroxy- or $1\alpha,25$-dihydroxy-vitamin $D_3$ amounts to about 5:1 to 1:5.

5. Preparations in accordance with claim 4, in which the weight ratio amounts to about 1:1.

6. Preparations in accordance with claims 1-5 containing $1\alpha,24,25$-trihydroxy-vitamin $D_3$ and $1\alpha,25$-dihydroxy-vitamin $D_3$.

7. Preparations in accordance with claims 1-6 containing 0.05-0.5 $\mu$g of each active substance per dosage unit.

8. Feedstuffs and feedstuff additives containing $1\alpha,25$-dihydroxy-vitamin$D_3$ in admixture with $1\alpha,24,25$-trihydroxy-vitamin $D_3$ or $1\alpha,25,26$-trihydroxy-vitamin $D_3$ or $25,26$-dihydroxy-vitamin $D_3$; or $1\alpha,24,25$-trihydroxy-vitamin $D_3$ and $24,25$-dihydroxy-vitamin $D_3$; or $1\alpha,25,26$-trihydroxy-vitamin $D_3$ and $25,26$-dihydroxy-vitamin $D_3$; and feedstuffs and feedstuff additives containing $1\alpha$-hydroxy-vitamin $D_3$ in admixture with $1\alpha,24,25$-trihydroxy-vitamin $D_3$ or $1\alpha,25,26$-trihydroxy-vitamin $D_3$.

9. The use of mixtures of $1\alpha$-dihydroxy-vitamin $D_3$ with $1\alpha,24,25$-trihydroxy-vitamin $D_3$ or $1\alpha,25,26$-trihydroxy-vitamin $D_3$ or $25,26$-dihydroxy-vitamin $D_3$; or $1\alpha,24,25$-trihydroxy-vitamin $D_3$ and $24,25$-dihydroxy-vitamin $D_3$; or $1\alpha,25,26$-trihydroxy-vitamin $D_3$ and $25,26$-dihydroxy-vitamin $D_3$; and mixtures of $1\alpha$-hydroxy-vitamin $D_3$ with $1\alpha,24,25$-trihydroxy-vitamin $D_3$ or $1\alpha,25,26$-trihydroxy-vitamin $D_3$ for the manufacture of pharmaceutical preparations, especially those for the treatment and prophylaxis of illnesses which are characterized by metabolic calcium and phosphate deficiency conditions, and for the manufacture of feedstuffs or feedstuff additives.

10. A process for the manufacture of the pharmaceutical preparations, feedstuffs and feedstuff additives of claims 1-8, characterized by mixing the active substances with conventional inert pharmaceutical adjuvants or carriers or with feedstuffs or with carriers which are conventional in animal nutrition.

## Revendications

1. Compositions pharmaceutiques contenant de la 1 alpha,25-dihydroxy-vitamine $D_3$ en mélange avec de la 1 alpha,24,25-trihydroxy-vitamine $D_3$ ou de la 1 alpha, 25,26-trihydroxy-vitamine $D_3$ ou de la 25,26-dihydroxy-vitamine $D_3$; ou de la 1 alpha,24,25-trihydroxy-vitamine $D_3$ et de la 24,25-dihydroxy-vitamine $D_3$; ou de la 1 alpha, 25,26-trihydroxy-vitamine $D_3$ et de la 25,26-dihydroxy-vitamine $D_3$; et oompositions pharmaceutiques contenant de la 1 alpha-hydroxy-vitamine $D_3$ en mélange avec de la 1 alpha,24,25-trihydroxy-vitamine $D_3$ ou de la 1 alpha,25, 26-trihydroxy-vitamine $D_3$; et des produits auxiliaires ou véhicules pharmaceutiques inertes usuels.

2. Compositions selon la revendication 1, contenant de la 1 alpha,24,25-trihydroxy-vitamine $D_3$ ou de la 1 alpha,25,26-trihydroxy-vitamine $D_3$ en mélange avec de la 1 alpha,25-dihydroxy-vitamine $D_3$.

3. Compositions selon la revendication 1, contenant de la 1 alpha,24,25-trihydroxy-vitamine $D_3$ en mélange avec de la 1 alpha-hydroxy-vitamine $D_3$ ou de la 1 alpha,25-dihydroxy-vitamine $D_3$.

4. Compositions selon la revendication 3, dans lesquelles les proportions relatives en poids entre la 1 alpha,24,25-trihydroxy-vitamine $D_3$ et de la 1 alpha-hydroxy- ou 1 alpha,25-dihydroxy-vitamine $D_3$ sont d'environ 5:1 à 1:5.

5. Compositions selon la revendication 4, dans lesquelles les proportions relatives en poids sont d'environ 1:1.

6. Compositions selon les revendications 1 à 5, contenant de la 1 alpha,24,25-trihydroxy-vitamine $D_3$ et de la 1 alpha,25-dihydroxy-vitamine $D_3$.

7. Compositions selon les revendications 1 à 6, contenant, par unité de dosage, 0,05 à 0,5 $\mu$g de chacune des substances actives.

8. Aliments pour animaux et additifs pour aliments pour animaux contenant de la 1 alpha,25-dihydroxy-vitamine $D_3$ en mélange avec de la 1 alpha,24,25-trihydroxy-vitamine $D_3$ ou de la 1 alpha,25,26-trihydroxy-vitamine $D_3$ ou de la 25,26-dihydroxy-vitamine $D_3$; ou de la 1 alpha,24,25-trihydroxy-vitamine $D_3$ et de la 24,25-dihydroxy-vitamine $D_3$ ; ou de la 1 alpha,25,26-trihydroxy-vitamine $D_3$ et de la 25,26-dihydroxy-vitamine $D_3$; et aliments pour animaux et additifs pour aliments pour animaux contenant de la 1 alpha-hydroxy-vitamine $D_3$ en mélange avec de la 1 alpha,24,25-trihydroxy-vitamine $D_3$ ou de la 1 alpha,25,26-trihydroxy-vitamine $D_3$.

9. Utilisation de mélanges de 1 alpha,25-dihydroxy-vitamine $D_3$ avec de la 1 alpha,24,25-trihydroxy-vitamine $D_3$ ou de la 1 alpha,25,26-trihydroxy-vitamine $D_3$ ou de la 25,26-dihydroxy-vitamine $D_3$; ou de la 1 alpha, 24,25-trihydroxy-vitamine $D_3$ et de la 24,25-dihydroxy-vitamine $D_3$; ou de la 1 alpha,25,26-trihydroxy-vitamine $D_3$ et de la 25,26-dihydroxy-vitamine $D_3$; et de mélanges de la 1 alpha-hydroxy-vitamine $D_3$ avec la 1 alpha,24,25-trihydroxy-vitamine $D_3$ ou de la 1 alpha,25,26-trihydroxy-vitamine $D_3$ pour la préparation de compositions pharmaceutiques, en particulier de compositions pharmaceutiques pour le traitement et la prophylaxie des maladies caractérisées par des états de carences métaboliques en calcium et phosphates, et pour la préparation d'aliments pour animaux et d'additifs pour aliments pour animaux.

10. Procédé de préparation de compositions pharmaceutiques, aliments pour animaux et additifs pour aliments pour animaux des revendications 1 à 8, caractérisé en ce que l'on mélange les substances actives avec des produits auxiliaires ou véhicules pharmaceutiques inertes usuels ou des aliments pour animaux ou dans des véhicules usuels pour l'alimentation animale.